# EUROPEAN PATENT SPECIFICATION

(11) **EP 1 060 722 B1**
(45) Date of publication and mention of the grant of the patent: **18.10.2006**
(21) Application number: 00111840.5
(22) Date of filing: 07.06.2000
(51) Int. Cl.: A61F 13/511, A61F 13/537

(54) **A unitized cover and absorbent transfer layer**
Einheitlich verbundene Schicht bestehend aus einer Deckschicht und einer flüssigkeitsleitenden Schicht
Couche composée d'une couche de transfert et d'une couche supérieure unifiés

(30) Priority: 18.06.1999 US 335825
(43) Date of publication of application: 20.12.2000
(73) Proprietor: McNEIL-PPC, Inc., Skillman, NJ 08558-9418 (US)
(72) Inventor: Lasko, Vincent O., New Egypt, NJ 08533 (US); O'Malley, Mary G., East Brunswick, NJ 08816 (US)
(74) Representative: Groening, Hans Wilhelm

(56) References cited:
- EP-A- 0 291 316
- GB-A- 2 281 212
- US-A- 4 307 721
- US-A- 4 377 615
- US-A- 4 650 481
- US-A- 4 704 112
- US-A- 5 178 945
- US-A- 5 273 596
- PHILIP BABCOCK GOVE: "webster's Third New International Dictionary of the English language unabridged" 1986, MERRIAM WEBSTER , SPRINGFIELD MASSACHUSETS USA

## Description

### Field of the Invention

The present invention relates to nonwoven webs useful as the body-contacting layer of absorbent articles. The webs are intended to transmit captured fluids away from the body and to an underlying absorbent core. The present invention also relates to absorbent articles employing such nonwoven webs as a unitized cover and transfer layer. The absorbent articles may be diapers, sanitary napkins, wound dressings, incontinence devices, and the like.

### Background of the Invention

Absorbent articles for managing discharged bodily-fluids are generally composed of a fluid permeable cover layer, a fluid impermeable barrier layer, and an absorbent core therebetween. The purpose of the cover layer is to contain the absorbent material and to transfer fluids to the absorbent core, while remaining relatively dry in use, thereby protecting against fluid leakage and increasing the comfort of the user. An additional layer may be employed in between to the absorbent core and the cover layer for improving the efficiency of fluid transfer, and for resisting re-transmission (rewet) of fluid from the absorbent core to the cover layer. The additional layer is commonly referred to as a transfer layer, acquisition layer, surge layer, or wicking layer (referred to as a transfer layer). The transfer layer in combination with a cover layer is often referred to as a composite cover. These are made from two separately manufactured webs bonded together.

Efficient fluid transfer and minimum rewet present conflicting requirements in the design of absorbent articles, with one or the other property typically being addressed to the detriment of the remaining property. One approach is to construct a transfer layer with relatively stiff, non-absorbent fibers, such as polyester fibers having deniers of 6 or greater. To ensure that an absorbent article employing such a transfer layer is comfortable to wear against the body, a cover made from apertured film or fine denier fibers is necessary to buffer the aggressiveness of the transfer layer. The stiffness of the fibers and the resulting large pore web in the transfer layer provides a spacer between the overlying cover and an underlying absorbent core in an effort to minimize rewet. This approach relies on a highly absorbent core to pull the fluid from the cover, through the transfer layer, and into the absorbent core. Although rewet may be improved with this approach, the time for fluid to penetrate the cover layer increases. The increase in fluid penetration time is due to both reliance on the absorbent core to draw the fluid from the cover, and the lack of capillary pressure between the relatively small pore cover layer and the larger pore transfer layer. If the cover layer feels clammy to a user, then discomfort occurs, with an extreme case impelling the user to replace the article before its useful life has terminated. Furthermore, the evolution of absorbent articles is gravitating towards thinner and thinner overall structures. As this happens, the first approach of a large pore size spacer fails to provide both efficient fluid transfer and rewet resistance. An example of this approach is disclosed by Baer et al. in U.S. Patent Number 5,728,081.

A second approach is to construct a transfer layer with a pore size that is smaller than that of an overlying cover. The pore size gradient from the cover to the transfer layer translates into higher capillary pressure, which results in captured fluid being drawn from the cover, without the reliance on an extremely absorbent core. The downside of this approach is that the transfer layer may not readily pass the fluid to the absorbent core after it has drawn the fluid form the cover layer. Any fluids that are held by the transfer layer yield the possibility of rewet. An example of this approach is disclosed by Meyer et al. in U.S. Patent Number 4,798,603.

Although transfer layers in general improve the performance of an absorbent article, in terms of fluid acquisition / transfer and resistance to rewetting the body-contacting surface, they add cost to the manufacture of the absorbent article. The added cost is a result of additional manufacturing equipment; handling, storing, and transportation of a greater number of material roll goods; and equipment / materials required to adhere the transfer layer to the remaining layers. Document US-A-4 377 615 relates to a nonwoven fabric used generally as an outer cover member of body exudates and treatment articles. The fabric comprises an upper layer containing hydrophobic fibers as a priciple element and a lower layer containing hydrophilic fibers and hydrophobic fibers. The denier of the fibers in the upper layer is finer than in the lower layer. The lower layer consists of a mixture of polypropylene fibers and rayon fibers.

It is an object of the present invention to provide a single nonwoven structure that provides improved cover layer and transfer layer properties. The single nonwoven structure employs a blend of thermoplastic fibers and absorbent fibers that offers a soft body-contacting surface, good fluid penetration, and good unidirectional fluid transfer from the body-contacting surface to an underlying absorbent core.

### Summary of the invention

The present invention relates to a unitized cover and transfer layer for an absorbent article comprising a blend of thermoplastic fibers and celluloic absorbent fibers according to claim 1 and 6 and to an absorbent article according to claim 8 and 9. There are two different denier thermoplastic fibers in the structure; the smaller of the two providing softness, and the larger of the two providing bulk. The absorbent fibers are present in an amount sufficient to efficiently draw fluid from the outer surface of the unitized cover and transfer layer, without competing with the absorbent core, thereby providing fast fluid penetration with minimal rewet.

In accordance with one embodiment of the present invention, there has now been provided a unitized nonwoven cover and transfer layer for an absorbent article, comprising: a first surface and a second surface opposite thereof for covering at least a portion of an absorbent core; thermoplastic fibers proximal the first surface; and a blend of thermoplastic fibers and absorbent fibers proximal the second surface, the absorbent fibers making up 20 weight percent or less of the blend. Preferably, the thermoplastic fibers proximal the first surface have a denier of 5 or less, and those proximal the second surface a denier of 6 or greater.

In accordance with a second embodiment of the present invention, there has now been provided an absorbent article comprising: a nonwoven web having a first surface and second surface thereof, thermoplastic fibers proximal the first surface, and a blend of thermoplastic fibers and absorbent fibers proximal the second surface, wherein the absorbent fibers make up 20 weight percent of less of the blend; and a liquid impermeable barrier layer adhered to at least a portion of the second surface.

### Brief Description of the Drawings

FIG. 1 is an end view of a unitized nonwoven cover and transfer layer provided by the present invention.
FIG.2 is a perspective view of an absorbent article provided by the present invention.

### Detailed Description of the Invention

The term "unitized", as used herein, refers to a single structural element. The single structural element may have two or more zones within its distinct outer surfaces exhibiting varying properties. Unitized does not encompass two or more separately manufactured structures that are laminated together.

The term "absorbent article", as used herein, refers to articles which absorb and contain body exudates. More specifically, the term refers to articles which are placed against or in proximity to the body of the wearer to absorb and contain the various exudates discharged from the body. The term "absorbent articles" is intended to include sanitary napkins, pantiliners, ultrathin napkins, incontinence pads, interlabial pads (and other articles worn in either the crotch region of a garment or attached directly to the body or simply held in place against the body with a garment), wound dressings, and the like.

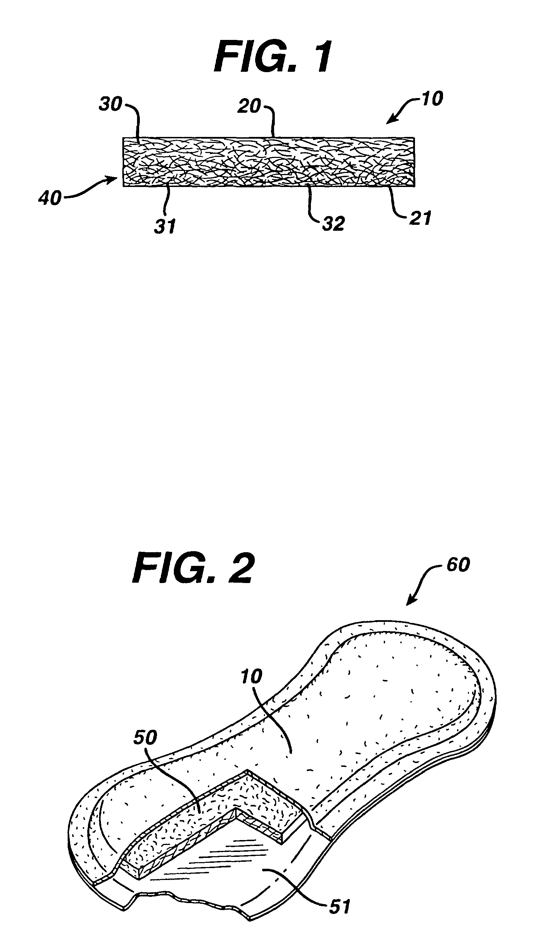

Referring to FIGS. 1 and 2, the present invention relates to a unitized cover and transfer layer **10** having a first surface **20** intended to face the body in use, and an opposing second surface **21**. Unitized layer **10** is preferably a nonwoven fibrous web, comprising at least 3 different fiber types. Proximal the first surface **20**, are thermoplastic fibers **30** having a denier of 5 or less, and preferably having a denier of 3 or less. As the denier of the thermoplastic fibers **30** decrease, the "softness" of the body-contacting surface **20** increases. Proximal the second surface **21** is a blend **40** of thermoplastic fibers **31** having a denier of 6 or greater, and absorbent fibers **32.** The absorbent fibers **32** are present in an amount sufficient to efficiently draw fluid from the first surface **20** of the unitized cover and transfer layer, without competing with an underlying absorbent core **50**, thereby providing fast fluid penetration with minimal rewet. The absorbent fibers **32** should comprise no greater than 20 weight percent of the blend **40** proximal the second surface **21**, and preferably no greater than 15 weight percent.

The unitized cover and transfer layer **10** preferably has a basis weight of about 20 to about 80 grams per square meter (gsm), and more preferably from about 30 to about 60 gsm. The blend **40** preferably makes up from about 50 to about 75 weight percent of the basis weight.

FIG. 2 depicts an absorbent article **60** having a liquid permeable unitized cover and transfer layer **10**, an absorbent core **50**, and a liquid impermeable barrier layer **51**. The absorbent article **60** has an hour-glass geometry, however other geometries may be employed.

Useful thermoplastic fibers **30** and **31** include polyolefin fibers such as polyethylene and polypropylene, polyester fibers, polyamide fibers (including nylon), polyacrylic fibers, and the like. Preferably at least a portion of the thermoplastic fibers **30** and **31** are bicomponent fibers, having either a sheath core or a side-by-side configuration. The two components generally have varying melting temperatures, with the lower melting temperature fiber being the sheath in a sheath / core configuration. The bicomponent thermoplastic fibers useful in the present invention include, but are not limited to polyethylene / polypropylene, polyethylene / polyester, polypropylene / polyester, copolyester / polyester.

If the thermoplastic fibers are not hydrophilic or hydrophobic per se, then they can be rendered hydrophilic or hydrophobic by appropriate treatments and / or finishes as known in the art. Referring again to FIG. 1, it may be useful for example to employ thermoplastic fibers **31** exhibiting hydrophilic properties proximal the second surface **21** to help the absorbent fibers **32** draw any captured fluid away from the body-contacting, first surface **20**. The thermoplastic fibers **30** proximal the first surface **20** however, would preferably not exhibit hydrophilic properties, in an effort to maintain a body-contacting surface having a "dry feeling", for optimal user comfort.

A representative, non-limiting list of cellulosic absorbent fibers 32 useful in the unitized cover and transfer layer **10**, includes cellulosic fibers such as cotton, rayon, cellulose acetate, wood pulp; and the like. Caution should be taken not to employ materials or amounts that would cause the unitized cover and transfer layer to compete in absorbency with an underlying absorbent core. The absorbent fibers **32** may be present homogeneously throughout the blend **40**, in discrete areas or in continuous or discontinuous concentration gradients. Preferably, the absorbent fibers **32** are rayon, and are uniformly present throughout the blend **40** proximal the second surface **21**.

The peripheral profile of thermoplastic and absorbent fibers may be of any shape, one example being trilobal. The fibers may also contain grooves, channels or bores; and may be pitted or perforated. The fibers may be crimped or not. The crimping may be two-dimensional, such as via a stuffer box, or three-dimensional, such as via hot water treatment.

Any process known in the art may be used to manufacture the unitized nonwoven structure **10**. It is preferably made with a process employing two cards, one containing the thermoplastic fibers **30**, and the second containing the blend **40** of thermoplastic fibers **31** and absorbent fibers **32**. A moving belt or screen carrier is passed underneath the first card, where the thermoplastic fibers **30** are deposited, and then passed underneath the second card, where the blend **40** is deposited. When at least a portion of the thermoplastic fibers **30** and **31** are bicomponent (described above), the nonwoven web created on the carrier may be thermally bonded by drawing hot air through the web and the carrier. The fibers are exposed to air heated to a temperature such that the lower melting temperature component, for example the sheath part, softens and begins to melt. Contact of this molten fiber with a second fiber will form a bond upon removal of the heated air. Contact between fibers can be achieved by the natural compression of gravity, the force of a moving stream of heated air against the fibers, and / or by a hold-down wire applying a compression force against the fibers. US Patent Number 4,548,856 discloses through-air bonding in more detail.

A second method of manufacturing the unitized cover and transfer layer of the present invention is through the use of transverse webber technology, as described in US Patent Numbers 4,931,357; 4,927,685; and 4,921,659. This method of manufacturing is particularly useful when short fibers are desired, such as cotton liners or wood pulp. The transverse webber technology may produce a nonwoven web with significant structural stability, thereby eliminating the necessity of binder material or bicomponent thermoplastic fibers. This technology also provides the possibility of controlled placement of the absorbent fibers **32** in discrete areas within the blend **40**. For example, in an effort to reduce leakage of an absorbent article, the absorbent fibers may be placed in concentrations along outer edges of the nonwoven web, a central portion, or combinations thereof.

A third method useful for manufacturing the unitized cover and transfer layer **10** includes the use of a single card for depositing the blend **40** on a carrier, and a spunbond apparatus for overlaying the blend **40** with the thermoplastic fibers **30**.

Absorbent articles employing a unitized cover and transfer layer, as described supra and shown in FIG. 2, are also contemplated by the present invention. The absorbent articles **60** of the present invention will also include at least a liquid impermeable barrier layer **51**, and preferably also an absorbent core **50** positioned between the unitized cover and transfer layer **10** and the liquid impermeable barrier layer **51**.

The liquid impermeable barrier layer **51** helps to prevent captured fluid from transferring to a user's body and / or clothing. The barrier layer can be of any flexible material that prevents the transfer of liquid, but does not necessarily prevent the passages of gases. Commonly used materials are polyethylene or polypropylene films.

Other materials that may be used as the liquid impermeable barrier layer **51** are films of polyesters, polyamides, ethylene vinyl acetate, polyvinyl chloride, polyvinylidene chloride, cellophane, nitrocellulose and cellulose acetate. Coextruded and laminated combinations of the foregoing, wherein such combinations are permitted by the chemical and physical properties of the film, may be used. Liquid impermeable reticulated foams and repellent treated papers may also be used.

Films that are barriers to liquids, but permit gases to transpire, i.e., "breathable barriers," may be used. These may be selected from polyurethane films and from microporous films in which microporosity is created by ionizing radiation or by leaching out of soluble inclusions using aqueous or nonaqueous solvents. Single or multiple layers of permeable films, fabrics and combinations thereof, that provide a tortuous path, and/or whose surface characteristics provide a liquid repellent surface to the penetration of liquids may also be used to provide such breathable barriers.

The absorbent core **50** provides the means for absorbing and retaining menses and other bodily fluids. The absorbent core is generally compressible, conformable, and non-irritating a user's skin. It can comprise any material used in the art for such purpose. The absorbent core **50** in the present invention may comprise either simple or complex absorbent structures that accept, transfer, distribute, store and retain fluid as well as prevent fluid from exiting the absorbent product. The absorbent core **50** may be composed of one or more layers of like, or dislike elemental features.

A representative, non-limiting list of materials that may be used in the absorbent core, includes natural materials such as comminuted wood pulp, creped cellulose wadding, hydrogel-forming polymer gelling agents, modified cross-linked cellulose fibers, capillary channel fibers, absorbent foams, absorbent sponges, synthetic staple fibers, polymeric fibers, peat moss, cotton, rayon, or any equivalent material or combinations of materials.

The polymeric gelling agents listed above may also be referred to as "absorbent gelling materials" or "superabsorbent materials." Polymeric gelling materials are those materials, which, upon contact with liquids such as water, or other body liquids, imbibe such liquids and thereby form hydrogels. In this manner, liquids discharged into the absorbent core **50** can be acquired and held by the polymeric gelling agent, thereby providing the articles herein with enhanced absorbency and / or improved liquid retention performance. The polymeric gelling agent that is optionally employed in the absorbent core **50** will generally comprise particles of a substantially water-insoluble, slightly cross-linked, partially neutralized, hydrogel-forming polymer material. The term "particles", as used herein, can refer to particles in any form, such as in the form of pellets, flakes, or fibers.

Attachment means may optionally be employed on one or more of the outwardly disposed surfaces of the article for adhering the articles to either the crotch region of a user's garment and / or directly to a user's body. Various mechanical and chemical (adhesives) attachment means are known in the art.

Materials which are water-soluble / dispersible and / or biodegradable may also be employed in one or more of the layer of the absorbent article, in an effort to provide a more flushable or less environmental persistent article. A non-limiting, representative list of such materials includes polyvinyl alcohol, polylactic acid, starch and starch based formulations, polyhydroxybutyrate, and combinations thereof.

The materials useful in one or more of the absorbent core, unitized cover and transfer layer, and liquid impermeable barrier layer above may have extensible / stretchable properties. The materials can be made extensible by performing a mechanical operation, such as pleating, corrugating, or ring rolling. In addition, the materials can be perforated or slit. The perforations or slits can vary in geometry and size, thereby providing extensibility in multiple directions if needed. The materials can also be inherently stretchable, such as polyethylene blended films available from Exxon, particularly film EXX-7. A more detailed disclosure of extensible articles, and methods of making such, is contained in US Patent Number 5,824,004.

There are a number of techniques useful to assemble all of the above mentioned layers of material into an absorbent article, including but not limited to the use of heat sealing, hook and fastener technology, construction adhesives, and ultrasonics. Preferably, construction adhesives are used in adhering the individual layers together. A variety of such materials are known in the art.

The absorbent articles of the present invention may be of any shape, such as rectangular or hour-glass. The article may one or more lateral extensions for wrapping around the crotch portion of a user's undergarment.

### Examples

Several 39.5 grams per square meter unitized cover and transfer layer samples were made with a two card and through-air bonding process, comprising 33 weight percent of 1.8 denier polyethylene / polyester bicomponent fibers proximal a first surface, and 67 weight percent of a blend of 10 denier polyethylene / polyester bicomponent fibers and rayon fibers proximal a second surface. The rayon fibers contributed to approximately 15 weight percent of the blend. The second surface of the unitized cover and transfer layer samples were placed adjacent three different absorbent cores for evaluating the performance of the samples. The fluid penetration time and rewet values for each are shown in Table 1 below. For comparison, multidenier (3 and 5) polypropylene (PP) covers in combination with alternative transfer layers are also shown.

The results in Table 1 illustrate that the unitized cover and transfer layer of the present invention provided (Examples 5, 10, and 15) a good balance between fluid penetration time and resistance to rewet. In comparison, the samples having a transfer layer made from 100 % absorbent fibers (Examples 3, 4, 8, 9, 13, and 14) provided relatively quick fluid penetration times, but were susceptible to rewet, while the samples having a transfer layer made from 100 % polyester fibers (Examples 1, 2, 6, 7, 11, and 12) provided acceptable resistance to rewet, but did not draw the fluid from the cover as quickly.

**TABLE 1**

| **Example** | **Absorbent Core** | **Cover** | **Transfer Layer** | **Fluid Penetration, sec.** | **Rewet, gms.** |
|---|---|---|---|---|---|
| 1 | 200 gsm pulp, 30 weight percent superabsorbent | 34 gsm multidenier PP | 30 gsm polyester | 109 | 0.50 |
| 2 | 200 gsm pulp, 30 weight percent superabsorbent | 34 gsm multidenier PP | 40 gsm polyester | 75 | 0.30 |
| 3 | 200 gsm pulp, 30 weight percent superabsorbent | 34 gsm multidenier PP | 65 gsm latex bonded pulp | 83 | 0.89 |
| 4 | 200 gsm pulp, 30 weight percent superabsorbent | 34 gsm multidenier PP | 2 x 65 gsm latex bonded pulp | 56 | 0.84 |
| 5 | 200 gsm pulp, 30 weight percent superabsorbent | Unitized Cover and Transfer Layer | | 63 | 0.45 |
| 6 | 250 gsm pulp, 40 weight percent superabsorbent | 34 gsm muitidenier PP | 30 gsm polyester | 86 | 0.19 |
| 7 | 250 gsm pulp, 40 weight percent superabsorbent | 34 gsm multidenier PP | 40 gsm polyester | 71 | 0.13 |
| 8 | 250 gsm pulp, 40 weight percent superabsorbent | 34 gsm multidenier PP | 65 gsm latex bonded pulp | 77 | 0.56 |
| 9 | 250 gsm pulp, 40 weight percent superabsorbent | 34 gsm multidenier PP | 2 x 65 gsm latex bonded pulp | 48 | 0.52 |
| 10 | 250 gsm pulp, 40 weight percent superabsorbent | Unitized Cover and Transfer Layer | | 59 | 0.14 |
| 11 | 300 gsm pulp, 40 weight percent superabsorbent | 34 gsm multidenier PP | 30 gsm polyester | 87 | 0.07 |
| 12 | 300 gsm pulp, 40 weight percent superabsorbent | 34 gsm multidenier PP | 40 gsm polyester | 74 , | 0.10 |
| 13 | 300 gsm pulp, 40 weight percent superabsorbent | 34 gsm multidenier PP | 65 gsm latex bonded pulp | 80 | 0.19 |
| 14 | 300 gsm pulp, 40 weight percent superabsorbent | 34 gsm multidenier PP | 2 x 65 gsm latex bonded pulp | 51 | 0.11 |
| 15 | 300 gsm pulp, 40 weight percent superabsorbent | Unitized Cover and Transfer Layer | | 64 | 0.08 |

### Test Methods

Fluid penetration time was measured by adding a 7 milliliter quantity of a synthetic body fluid or a saline solution to a contained area on an outwardly disposed surface of an article, starting the time measurement when the fluid first contacted the article's outer surface and stopping the time measurement when any portion of the surface in the contained area became visible. The contained area was defined by overlaying the article with a plate having an oval orifice therein. The plate had a length of approximately 25 centimeters, a width of approximately 7.5 centimeters, and thickness of approximately 1.3 centimeters. The oval had a central width of 3.8 centimeters and a central height of 1.9 centimeters. The synthetic fluid was poured into the orifice from a height of approximately 2.5 to 7.5 centimeters above the orifice plate, keeping the orifice as full as possible without overflowing the face of the plate.

Rewet was measured by covering the contained wetted area, as formed from the fluid penetration test above, with a layered absorbent structure, placing it under a specific load for a specific time, and then calculating the weight of fluid absorbed by the absorbent structure. The rewet test began 5 minutes after the 7 milliliter quantity of fluid penetrated the outer surface of the article. Two NUGAUZE general use sponges (available from Johnson & Johnson Hospital Services), having a 4 inch width and length, and comprising 4 layers were folded once down a central axis and staked on top of one another with the creased edges placed opposing each other, thereby creating a layered absorbent structure approximately 2 inches by 4 inches by 16 plys. The 16 ply layered absorbent structure was concentrically placed over the wetted area of the article. Weights were then placed onto the layered absorbent structure, resulting in a pressure of 0.6 pounds per square inch. The weights and staked sponges were removed from the wetted area after 3 minutes. The sponges and any absorbed fluid were weighed, thereby defining a final weight. The amount of fluid absorbed by the structure (released by the article) was calculated by subtracting a pre-weight of the staked sponges from the final weight

## Claims

1. A unitized nonwoven cover and transfer layer (10) for an absorbent article, comprising:
a) a first surface (20) and a second surface (21) opposite thereof for covering at least a portion of an absorbent core;
b) thermoplastic fibers (30) proximal the first surface (20);
**characterized by**
c) a blend (40) of thermoplastic fibers (31) and cellulosic absorbent fibers (32) proximal the second surface (21), the cellulosic absorbent fibers (32) making up 20 weight percent or less of the blend (40).

2. The unitized nonwoven cover and transfer layer (10) of claim 1 wherein the nonwoven has a basis weight and the blend (40) of thermoplastic and cellulosic absorbent fibers (31, 32) proximal the second surface (21) make up from about 50 to about 75 weight percent of the basis weight.

3. The unitized nonwoven cover and transfer layer (10) of claim 2 wherein the basis weight is from about 20 to about 80 grams per square meter.

4. The unitized nonwoven cover and transfer layer (10) of claim 1 wherein the thermoplastic fibers (30) proximal the first surface (20) have a denier of 5 or less.

5. The unitized nonwoven cover and transfer layer (10) of claim 1 wherein the thermoplastic fibers (31) proximal the second surface (21) have a denier of 6 or greater.

6. A unitized nonwoven cover and transfer layer (10) for an absorbent article, comprisiing:
a) a first surface (20) and a second surface (21) opposite thereof for covering at least a portion of an absorbent core;
**characterized by**
- b) thermoplastic fibers (30) having a denier of 3 or less proximal the first surface (20); and
c) a blend (40) of thermoplastic fibers (31) and cellulosic absorbent fibers (32) proximal the second surface (21), wherein the cellulosic absorbent fibers (32) make up 20 weight percent or less of the blend (40) and the thermoplastic fibers (31) have a denier of 6 or greater.

7. The unitized nonwoven cover and transfer layer (10) of claim 6 wherein the nonwoven has a basis weight and the blend (40) of thermoplastic and absorbent fibers (31, 32) proximal the second surface make up from about 50 to about 75 weight percent of the basis weight.

8. An absorbent article (60), comprising a unitized nonwoven cover and transfer layer (10) according to claim 1; a liquid impermeable barrier layer (51); and an absorbent core (50) therebetween.

9. An absorbent article (60) comprising:
a) a nonwoven web having a first surface (20) and a second surface (21) opposite thereof, thermoplastic fibers (30) proximal the first surface (20), **characterized by** a blend (40) of thermoplastic fibers (31) and cellulosic absorbent fibers (32) proximal the second surface (21), wherein the cellulosic absorbent fibers (32) make up 20 weight percent or less of the blend (40); and
b) a liquid impermeable barrier layer (51) adhered to at least a portion of the second surface (21).

## Patentansprüche

1. Modularisierte Deck- und Transfervliesstoffschicht (10) für einen absorbierenden Artikel, umfassend:
a) eine erste Oberfläche (20) und eine zweite, dieser gegenüberliegende Oberfläche (21) zum Abdecken zumindest eines Teils eines absorbierenden Kerns;
b) thermoplastische Fasern (30) proximal zur ersten Oberfläche (20);
**gekennzeichnet durch**
c) eine Mischung (40) aus thermoplastischen Fasern (31) und absorbierenden Cellulosefasern (32) proximal zur zweiten Oberfläche (21), wobei die absorbierenden Cellulosefasern (32) 20 Gewichtsprozent oder weniger der Mischung (40) ausmachen.

2. Modularisierte Deck- und Transfervliesstoffschicht (10) nach Anspruch 1, **dadurch gekennzeichnet, daß** der Vliesstoff ein Basisgewicht aufweist und daß die Mischung (40) aus thermoplastischen und absorbierenden Cellulosefasern (31, 32) proximal zur zweiten Oberfläche (21) etwa 50 bis etwa 75 Gewichtsprozent des Basisgewichts ausmacht.

3. Modularisierte Deck- und Transfervliesstoffschicht (10) nach Anspruch 2, **dadurch gekennzeichnet, daß** das Basisgewicht von etwa 20 bis etwa 80 Gramm pro Quadratmeter beträgt.

4. Modularisierte Deck- und Transfervliesstoffschicht (10) nach Anspruch 1, **dadurch gekennzeichnet, daß** die proximal zur ersten Oberfläche (20) gelegenen thermoplastischen Fasern (30) ein Denier von 5 oder weniger aufweisen.

5. Modularisierte Deck- und Transfervliesstoffschicht (10) nach Anspruch 1, **dadurch gekennzeichnet, daß** die proximal zur zweiten Oberfläche (21) gelegenen thermoplastischen Fasern (31) ein Denier von 6 oder höher aufweisen.

6. Modularisierte Deck- und Transfervliesstoffschicht (10) für einen saugfähigen Artikel, umfassend:
a) eine erste Oberfläche (20) und eine zweite, dieser gegenüberliegende Oberfläche (21) zum Abdecken zumindest eines Teils eines saugfähigen Kerns;
**gekennzeichnet durch**
b) thermoplastische Fasern (30) mit einem Denier von 3 oder weniger proximal zur ersten Oberfläche (20) und
c) eine Mischung (40) aus thermoplastischen Fasern (31) und absorbierenden Cellulosefasern (32) proximal zur zweiten Oberfläche (21), wobei die absorbierenden Cellulosefasern (32) 20 Gewichtsprozent oder weniger der Mischung (40) ausmachen und wobei die thermoplastischen Fasern (31) ein Denier von 6 oder höher aufweisen.

7. Modularisierte Deck- und Transfervliesstoffschicht (10) nach Anspruch 6, **dadurch gekennzeichnet, daß** der Vliesstoff ein Basisgewicht aufweist und daß die Mischung (40) aus thermoplastischen und saugfähigen Fasern (31, 32) proximal zur zweiten Oberfläche etwa 50 bis etwa 75 Gewichtsprozent des Basisgewichts ausmachen.

8. Saugfähiger Artikel (60), umfassend eine modularisierte Deck- und Transfervliesstoffschicht (10) nach Anspruch 1, eine flüssigkeitsundurchlässige Barriereschicht (51) und einen dazwischen befindlichen absorbierenden Kern (50).

9. Saugfähiger Artikel (60), umfassend:
a) einen Vliesstoff mit einer ersten Oberfläche (20) und eine zweite, dieser gegenüberliegende Oberfläche (21), thermoplastischen Fasern (30) proximal zur ersten Oberfläche (20), **gekennzeichnet durch** eine Mischung (40) aus thermoplastischen Fasern (31) und absorbierenden Cellulosefasern (32) proximal zur zweiten Oberfläche (21), wobei die absorbierenden Cellulosefasern (32) 20 Gewichtsprozent oder weniger der Mischung (40) ausmachen, und
b) eine flüssigkeitsundurchlässige Barriereschicht (51), die zumindest mit einem Teil der zweiten Oberfläche (21) verbunden ist.

## Revendications

1. Couche de transfert et couche supérieure non tissée unifiées (10) pour un article absorbant, comprenant :
a) une première surface (20) et une seconde surface (21) à l'opposé de celle-ci, destinées à recouvrir au moins une partie d'un noyau absorbant ;
b) des fibres thermoplastiques (30) proximales à la première surface (20) ;
**caractérisées par**
c) un mélange (40) de fibres thermoplastiques (31) et de fibres absorbantes cellulosiques (32) proximal à la seconde surface (21), les fibres absorbantes cellulosiques (32) constituant jusqu'à 20 pour cent en poids du mélange (40) ou moins.

2. Couche de transfert et couche supérieure non tissée unifiées (10) selon la revendication 1, dans lesquelles le non tissé présente une masse surfacique et le mélange (40) de fibres thermoplastiques et absorbantes cellulosiques (31, 32) proximal à la seconde surface (21) constitue d'environ 50 à environ 75 pour cent en poids de la masse surfacique.

3. Couche de transfert et couche supérieure non tissée unifiées (10) selon la revendication 2, dans lesquelles la masse surfacique est d'environ 20 à environ 80 grammes par mètre carré.

4. Couche de transfert et couche supérieure non tissée unifiées (10) selon la revendication 1, dans lesquelles les fibres thermoplastiques (30) proximales à la seconde surface (20) présentent un denier inférieur ou égal à 5.

5. Couche de transfert et couche supérieure non tissée unifiées (10) selon la revendication 1, dans lesquelles les fibres thermoplastiques (31) proximales à la première surface (21) présentent un denier supérieur ou égal à 6.

6. Couche de transfert et couche supérieure non tissée unifiées (10) pour un article absorbant, comprenant :
a) une première surface (20) et une seconde surface (21) à l'opposé de celle-ci, destinées à recouvrir au moins une partie d'un noyau absorbant ;
**caractérisées par**
b) des fibres thermoplastiques (30) présentant un denier inférieur ou égal à 3 et proximales à la première surface (20) ; et
c) un mélange (40) de fibres thermoplastiques (31) et de fibres absorbantes cellulosiques (32) proximal à la seconde surface (21), dans lequel les fibres absorbantes cellulosiques (32) constituent au maximum 20 pour cent en poids du mélange (40) et les fibres thermoplastiques (31) présentent un denier supérieur ou égal à 6.

7. Couche de transfert et couche supérieure non tissée unifiées (10) selon la revendication 6, dans lesquelles le non tissé présente une masse surfacique et le mélange (40) de fibres thermoplastiques et absorbantes (31, 32) proximal à la seconde surface constitue d'environ 50 à environ 75 pour cent en poids de la masse surfacique.

8. Article absorbant (60), comprenant une couche de transfert et une couche supérieure non tissée unifiées (10) selon la revendication 1 ; une couche barrière étanche au liquide (51) ; et un noyau absorbant (50) entre celles-ci.

9. Article absorbant (60), comprenant :
a) un voile non tissé ayant une première surface (20) et une seconde surface (21) à l'opposé de celle-ci, des fibres thermoplastiques (30) proximales à la première surface (20), **caractérisé par** un mélange (40) de fibres thermoplastiques (31) et de fibres absorbantes cellulosiques (32) proximal à la seconde surface (21), dans lequel les fibres absorbantes cellulosiques (32) constituant au maximum 20 pour cent en poids du mélange (40) ; et
b) une couche barrière étanche au liquide (51) collée à au moins une partie de la seconde surface (21).
